# EUROPÄISCHE PATENTSCHRIFT

(11) **EP 3 468 503 B1**
(45) Veröffentlichungstag und Bekanntmachung des Hinweises auf die Patenterteilung: **08.04.2026**
(21) Anmeldenummer: 17728038.5
(22) Anmeldetag: 12.05.2017
(51) Int. Cl.: A61F 2/00

(54) **MEDIZINISCHE EINRICHTUNG ZUM ABSPERREN EINES KÖRPERKANALS**
MEDICAL ARRANGEMENT FOR SHUTTING OFF A BODY CHANNEL
DISPOSITIF MÉDICAL POUR FERMER UN CANAL CORPOREL

(30) Priorität: 14.06.2016 AT 2942016
(43) Veröffentlichungstag der Anmeldung: 17.04.2019
(73) Patentinhaber: A.M.I. Agency for Medical Innovations GmbH, 6800 Feldkirch (AT)
(72) Erfinder: HOHLRIEDER, Martin, 6840 Götzis (AT); JABLONOWSKI, Marc, 6923 Lauterach (AT)
(74) Vertreter: Torggler & Hofmann Patentanwälte - Rankweil
(86) Internationale Anmeldenummer: PCT/AT2017/000036
(87) Internationale Veröffentlichungsnummer: WO 2017/214643

(56) Entgegenhaltungen:
- WO-A2-01/54615
- US-A- 4 721 509
- US-A- 4 784 660
- US-A1- 2009 248 109
- US-A1- 2010 211 175

## Beschreibung

Die Erfindung bezieht sich auf eine medizinische Einrichtung zum Absperren eines Körperkanals, umfassend ein Bandteil, das um das den Körperkanal umgebende Körpergewebe legbar ist und zu einem eine Durchtrittsöffnung für das Körpergewebe umschließenden Ring schließbar ist und das eine Hohlkammer aufweist, die einen Teil eines Aufnahmeraums der Einrichtung zur Aufnahme von Arbeitsfluid darstellt, und eine Pumpeinheit zum Fördern des Arbeitsfluids, wobei durch Einbringen des Arbeitsfluids in die Hohlkammer die Durchtrittsöffnung verkleinerbar ist, wobei die Einrichtung im Weiteren einen Expansionskörper mit einer Expansionskammer aufweist, und durch Einbringen eines vom Arbeitsfluid getrennten Hilfsfluid in die Expansionskammer die Expansionskammer vergrößerbar ist.

Medizinische Einrichtungen zum Absperren eines Körperkanals werden u.a. als künstliche (Schließ-)Muskeln zur Unterstützung oder zum Ersatz von geschwächten natürlichen Muskeln im menschlichen oder tierischen Körper eingesetzt. Beispiele für Einsatzbereiche derartiger Einrichtungen sind Analbänder zum Verschließen eines, gegebenenfalls künstlichen, Anus und künstliche Schließmuskel zur Behandlung von Inkontinenz zum Verschließen der Urethra (=Harnröhre). Weitere Einsatzgebiete sind z.B. Bänder zum Verschließen eines Gangs für Gallenflüssigkeit. Das Bandteil derartiger medizinischer Einrichtungen wird auch als Cuff, Manschette oder artifizieller Sphinkter bezeichnet.

Die Hohlkammer des Bandteils kann vom Benutzer bei Bedarf entleert werden, um die Querschnittsfläche der Durchtrittsöffnung zu vergrößern und im Körperkanal enthaltene Stoffe und/oder Flüssigkeiten passieren zu lassen. Z.B. bei der Anwendung als künstlicher Schließmuskel für die Urethra erfolgt häufig ein anschließendes automatisches Verschließen des Körperkanals durch Rückpumpen von Arbeitsfluid (evtl. über ein Drosselventil) in die Hohlkammer des Bandteils. Eine Pumpe zum Pumpen von Arbeitsfluid wird bei einem solchen künstlichen Harnsphinkter für männliche Patienten üblicherweise in das Skrotum implantiert. Das Pumpen von Arbeitsfluid aus der Hohlkammer kann dann durch Druck auf einen flexiblen Teil der Pumpe erfolgen. Das Rückpumpen von Arbeitsfluid in die Hohlkammer kann durch ein federelastisches Element der Pumpe erfolgen. Die Durchtrittsöffnung der medizinischen Einrichtung kann häufig auch durch eine bewusste Handlung des Benutzers wieder verkleinert werden, also durch eine manuelle Betätigung der Pumpe.

Z.B. aus der US 2014/0364686 A1 sind mehrere Ausführungsbeispiele medizinischer Einrichtungen in Form von Urethrasphinktern bekannt, welche ein Bandteil mit zwei Hohlkammern aufweisen. Eine Zwischenwand, welche die beiden Hohlkammern voneinander trennt, weist ein Ventil auf, welches einen Fluidaustausch zwischen den Hohlkammern ermöglicht. Mittels einer Pumpe kann das Fluid zum Öffnen des Körperkanals von einer der Hohlkammern in die andere Hohlkammer umgeschichtet werden. Weitere Beispiele medizinischer Einrichtungen gehen aus der US 2010/0211175 A1 und der US 2009/0248109 A1 hervor.

Problematisch bei medizinischen Einrichtungen zum Verengen oder Absperren eines Körperkanals ist, dass diese aufgrund des von der Einrichtung ausgeübten Druckes auf das Körpergewebe eine Erosion des Körpergewebes hervorrufen können. Deshalb wird der Druck des Arbeitsfluids in der Hohlkammer in der Regel so gewählt, dass die Erosion von Körpergewebe möglichst gering gehalten werden kann und gleichzeitig noch ein zuverlässiges Absperren des Körperkanals erreicht wird.

Bei einer Anspannung der Bauchmuskeln, wie dies z.B beim Treppensteigen, Heben von Lasten, Niesen, Husten, Lachen, meist unwillkürlich auftritt, erfolgt eine kurzzeitige intraabdominelle (= innerhalb des Bauchraums) Erhöhung des Körperinnendrucks. Dabei wirken die kurzzeitigen Druckspitzen insbesondere auf die im Bauchraum angeordneten inneren (Hohl-)Organe, z.B. Harnblase und Darm.

Dadurch nimmt der Druck in den (Hohl-)Organen zu, was zu einem Austritt von Stoffen und/oder Flüssigkeit durch den vom Bandteil abgesperrten Abschnitt des Körperkanals führen kann. Diese Art der Inkontinenz wird auch als Stressinkontinenz oder Belastungsinkontinenz bezeichnet.

Aus der US 5,478,305 A geht eine medizinische Einrichtung zur Behandlung von Harn- oder Stuhlinkontinenz hervor. Das in dieser Schrift als Cuff bezeichnete Bandteil ist aus Silikon hergestellt. Durch die Befüllung des Cuffs mit Arbeitsfluid steigt der Druck im Hohlraum des Cuffs an, wobei die dadurch bewirkte Verschiebung eines inneren Abschnitts des Cuffs in Richtung zur Längsmittelachse hin den Körperkanal verschließt. Zwischen zwei Teilen einer Verbindungsleitung, welche eine Pumpe mit dem Cuff fluidleitend verbindet, ist ein flexibler Ballon (="Stressballon") aus Silikon angeordnet. Der Hohlraum des Stressballons ist mit Arbeitsfluid befüllt. Eine kurzzeitige Erhöhung des Körperinnendrucks bewirkt eine Volumenverkleinerung des Hohlraums des Stressballons, wodurch Arbeitsfluid in den Cuff verdrängt wird. Durch die kurzzeitige Erhöhung des Drucks des Arbeitsfluids kann der Körperkanal auch während eines Stressereignisses abgesperrt gehalten und eine Leckage verhindert werden. Verringert sich der Körperinnendruck anschließend wieder, so vergrößert sich das Volumen des Hohlraums des Stressballons und der Druck des Arbeitsfluid nimmt ab, wobei Arbeitsfluid aus dem Cuff herausströmt. Wird der Druck des Arbeitsfluids im Cuff zum Schließen des Körperkanals erhöht, so erhöht sich auch das Volumen des Ballons, sodass mittels der Pumpe eine entsprechend große Flüssigkeitsmenge verschoben werden muss.

Aus der US 4,784,660 A geht eine medizinische Einrichtung der eingangs genannten Art mit einer Pumpeinheit hervor, die eine Arbeitsfluidkammer und eine Hilfsfluidkammer aufweist. Die Arbeitsfluidkammer ist mit einem Arbeitsfluid und die Hilfsfluidkammer mit einem Hilfsfluid befüllbar bzw. befüllt. Durch Hinzufügen von Hilfsfluid in die Hilfsfluidkammer erfolgt eine Volumenvergrößerung der Hilfsfluidkammer und zugleich eine Volumenverkleinerung der Arbeitsfluidkammer, wodurch eine Erhöhung des Druckes des Arbeitsfluids bewirkt wird. Dadurch ist eine Zusatzkraft auf die, durch ein mittels des Arbeitsfluids befüllbares Bandteil der medizinischen Einrichtung geführte, Urethra aufbringbar. Eine ähnliche medizinische Einrichtung geht auch aus der US 4,721,509 A hervor.

Aufgabe der Erfindung ist es, eine vorteilhafte Einrichtung der eingangs genannten Art bereitzustellen, welche zur Behandlung von Stressinkontinenz eingesetzt werden kann.

Erfindungsgemäß gelingt dies durch eine Einrichtung mit den Merkmalen des Anspruchs 1.

Die Einrichtung gemäß der Erfindung weist einen Expansionskörper mit einer Expansionskammer auf, wobei der Expansionskörper an einer der Durchtrittsöffnung zugewandten Seite des Bandteils am Bandteil angeordnet ist. Durch Einbringen des vom Arbeitsfluid getrennten Hilfsfluids in die Expansionskammer ist die Expansionskammer vergrößerbar. Die Expansionskammer ist somit unabhängig vom Arbeitsfluid mit dem Hilfsfluid befüllbar.

Beim Auftreten eines Stressereignisses, z.B. hervorgerufen durch einen Anstieg des Körperinnendrucks bei einem Hustenanfall, erfolgt eine Vergrößerung der Expansionskammer, wodurch eine Zusatzkraft auf das durch die Durchtrittsöffnung geführte Körpergewebe aufgebracht werden kann. Der Körperkanal wird somit auch während eines Stressereignisses zuverlässig abgesperrt. Während eines Stressereignisses liegt der Körperinnendruck (= Umgebungsdruck) jedenfalls über dem Atmosphärendruck.

Das Hilfsfluid ist von dem Arbeitsfluid günstigerweise vollständig getrennt, d.h. dass kein Flüssigkeitsaustausch zwischen dem Hilfsfluid und dem Arbeitsfluid stattfindet.

Durch das Vorsehen von zwei unabhängigen Fluidsystemen können zusätzliche Möglichkeiten zur Auslegung der medizinischen Einrichtung erhalten werden.

Die Vergrößerung des Volumens des Expansionskörpers durch Einfüllen von Hilfsfluid in die Expansionskammer erfolgt insbesondere durch Dehnen oder Entfalten des Expansionskörpers.

Es kann vorgesehen sein, dass der Expansionskörper elastisch dehnbar ist. In anderen Ausführungsformen kann der Expansionskörper aber auch aus einem zumindest im Wesentlichen undehnbaren Material ausgebildet sein. So könnte ein beidseitig verschlossener Balg vorgesehen sein, welcher sich durch Einfüllen von Hilfsfluid in die Expansionskammer ausdehnt. Es ist grundsätzlich auch denkbar und möglich, dass die Volumenänderung des Expansionskörpers durch Verschieben mindestens einer verschiebbaren Wand des Expansionskörpers erfolgt. Die verschiebbare Wand könnte z.B. ein Kolben sein, welcher durch Einfüllen von Hilfsfluid in die Expansionskammer bewegbar ist.

Als Aufnahmeraum für das Arbeitsfluid wird in dieser Schrift der gesamte Innenhohlraum der Einrichtung bezeichnet, in welchem sich im Betrieb der Einrichtung Arbeitsfluid befindet.

Die Pumpeinheit weist günstigerweise ein Pumpteil auf, das einen Innenraum zur Aufnahme von Arbeitsfluid aufweist, dessen Volumen mittels eines Antriebs veränderbar ist.

Mittels der Pumpeinheit kann Arbeitsfluid in die Hohlkammer des Bandteils eingebracht werden. Durch Verschieben eines der Durchtrittsöffnung zugewandten Innenabschnitts des Bandteils in Richtung hin zu einer Längsmittelachse der Durchtrittsöffnung kann der Körperkanal abgesperrt werden.

Bei der Ausgestaltungsform gemäß der Erfindung, ist der Expansionskörper an einer der Durchtrittsöffnung zugewandten Seite des Bandteils am Bandteil angeordnet, also am Innenabschnitt des Bandteils, und ein Befüllen der Expansionskammer mit Hilfsfluid wirkt durch die dadurch bewirkte Vergrößerung des Volumens der Expansionskammer direkt komprimierend auf das den Körperkanal umgebende Körpergewebe ein, um ein zuverlässiges Absperren des Körperkanals während eines Stressereignisses zu gewährleisten.

Das Befüllen der Expansionskammer könnte grundsätzlich mit einer sensorgesteuerten Hilfsfluidpumpe erfolgen, wobei eine Druckerhöhung im Körperinneren von einem Drucksensor erfasst und eine davon abhängige Aktivierung der Hilfsfluidpumpe erfolgen könnte. In einer bevorzugten Ausgestaltungsform weist die Einrichtung jedoch einen Hilfsfluidbehälter mit einer Speicherkammer für das Hilfsfluid auf, deren Volumen in Abhängigkeit vom Umgebungsdruck veränderbar ist. Der Hilfsfluidbehälter ist über eine Hilfsfluidleitung mit der Expansionskammer verbunden.

Der Hilfsfluidbehälter ist günstigerweise im Körper, insbesondere im Bauchraum, implantierbar, wobei der im Körper wirkende Körperinnendruck auf den Hilfsfluidbehälter einwirkt. Günstigerweise entspricht dann der Druck des Hilfsfluid dem Körperinnendruck. Beim Anspannen von Bauchmuskeln während eines Stressereignisses, ändert sich das Volumen der Speicherkammer des Hilfsfluidbehälters in Abhängigkeit vom Körperinnendruck. Dabei wird Hilfsfluid aus der Speicherkammer ausgestoßen und durch die Hilfsfluidleitung hindurch in die Expansionskammer geleitet. Zur Beförderung von Hilfsfluid ist somit keine zusätzliche Pumpe nötig.

Günstigerweise weist der Hilfsfluidbehälter eine die Speicherkammer begrenzende flexible Wand auf. Unter flexibel wird in diesem Zusammenhang eine verbiegbare Wand angesehen, welche aber nicht zwingendermaßen dehnbar sein muss. Vorzugsweise ist die flexible Wand im Gegenteil zumindest im Wesentlichen undehnbar ausgebildet. Beim Befüllen des Hilfsfluidbehälters erfolgt dann also keine Materialdehnung, d.h. dass das Volumen der Speicherkammer nur durch Ausfalten der flexiblen Wand vergrößerbar ist. Das Volumen der Speicherkammer ist dabei günstigerweise auf ein maximales Speichervolumen der Speicherkammer begrenzt.

In einer vorteilhaften Ausgestaltungsform ist vorgesehen, dass die die Speicherkammer begrenzende flexible Wand biegeschlaff ausgebildet ist. D.h., dass die Wand nicht von selbst (=ohne Einwirken einer äußeren Kraft) zu einer früheren Form zurückkehrt. Bei einer biegeschlaff ausgebildeten Wand könnte der Hilfsfluidbehälter auch als formlabil bezeichnet werden.

In einer anderen Ausgestaltungsform könnte als Hilfsfluidbehälter beispielsweise auch ein beidseitig verschlossener Balg vorgesehen sein, wobei das Volumen der Speicherkammer durch Ausdehnung des Balgs vergrößerbar ist.

Grundsätzlich wäre es aber auch denkbar, dass der Hilfsfluidbehälter zumindest eine verschiebbare Wand aufweist, z.B. nach Art eines Kolbens.

Vorteilhafterweise ist vorgesehen, dass das Volumen der Expansionskammer bei einem im Vergleich zum Druck des Hilfsfluids unter einem höheren Druck stehenden Arbeitsfluid zumindest im Wesentlichen gleich Null ist. Wenn im Freigabezustand das Volumens des Expansionskörpers im Wesentlichen gleich Null oder relativ gering ist, so muss beim Verstellen des Bandteils vom Freigabezustand in den Absperrzustand kein oder nur wenig Hilfsfluid von der Expansionskammer in die Speicherkammer des Hilfsfluidbehälters gefördert werden. Dadurch kann die Menge an zu verschiebendem Arbeitsfluid minimiert werden. Im Fall, dass die medizinische Einrichtung eine elektrisch betriebene Pumpeinrichtung aufweist, kann dadurch eine Energieeinsparung realisiert werden.

Weitere Vorteile und Einzelheiten der Erfindung werden im Folgenden anhand der beiliegenden Zeichnungen erläutert. In diesen zeigen:
Fig. 1 eine schematische Darstellung einer als künstlicher Urethrasphinkter ausgebildeten, nicht erfindungsgemäßen, Einrichtung, in einem Freigabezustand des Bandteils, in welchem der Harnleiter geöffnet ist;
Fig. 2 eine Darstellung analog Fig. 1 in einem Absperrzustand des Bandteils, in welchem der Harnleiter geschlossen ist;
Fig. 3 eine Darstellung analog Fig. 1 in einem Stresszustand des Bandteils, in welchem eine Zusatzkraft auf den Harnleiter ausgeübt wird;
Fig. 4 und 5 Schrägsichten des freien, also nicht implantierten, Bandteils der Einrichtung in einem geöffneten und geschlossenen Zustand, und zwar entsprechend dem Freigabezustand;
Fig. 6 einen Längsmittelschnitt (parallel zur Längsmittelachse der Durchtrittsöffnung und diese durchsetzend) durch das Bandteil im Zustand gemäß Fig. 5;
Fig. 7 einen Längsmittelschnitt analog Fig. 6, aber im Absperrzustand des Bandteils;
Fig. 8 eine schematische Darstellung einer erfindungsgemäßen Ausführungsform eines künstlichen Urethrasphinkters in einem Absperrzustand des Bandteils, in welchem der Harnleiter geschlossen ist;
Fig. 9 eine Darstellung analog Fig. 8 in einem Stresszustand des Bandteils, in welchem eine Zusatzkraft auf den Harnleiter ausgeübt wird;
Fig. 10 eine schematische Darstellung einer Variante des Bandteils gemäß der erfindungsgemäßen Ausführungsform in einem Absperrzustand des Bandteils, in welchem der Harnleiter geschlossen ist, und
Fig. 11 eine Darstellung analog Fig. 10 in einem Stresszustand des Bandteils, in welchem eine Zusatzkraft auf den Harnleiter ausgeübt wird.

Ein Bandteil 1 der Medizinischen Einrichtung ist ringförmig um das den Körperkanal umgebende Körpergewebe 2, hier die Harnröhre, legbar. Das Bandteil 1 weist eine Hohlkammer 3 auf, die sich in Richtung der Längserstreckung des Bandteils 1 erstreckt, in den Ausführungsbeispielen im Wesentlichen über die gesamte Länge des Bandteils 1. Das Bandteil 1 ist somit schlauchartig ausgebildet mit beidseitig verschlossenen Enden.

An den beiden Enden des Bandteils 1 sind ein erstes und ein zweites Verschlussteil 6, 7 angeordnet. Das erste Verschlussteil 6 besitzt eine Einstecköffnung 6a, in welche eine Zunge 7a des zweiten Verschlussteils 7 einsteckbar und darin verrastbar ist.

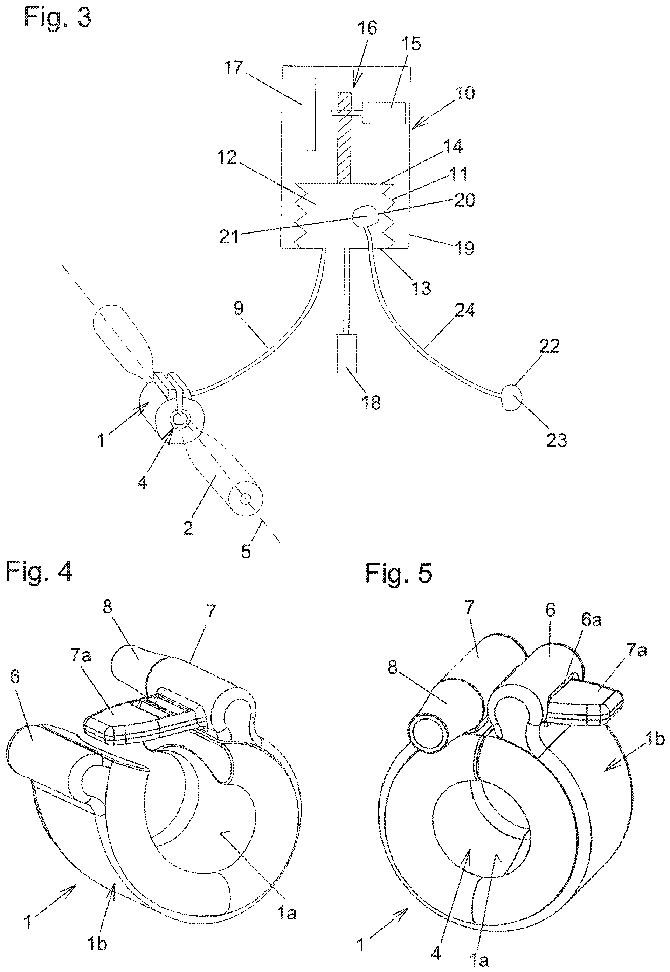

Die Verschlussteile 6, 7 bilden somit einen Verschluss, mit welchem das Bandteil 1 zu einem Ring, insbesondere Kreisring, geschlossen werden kann, vgl. Fig. 5. Im geschlossenen Zustand umschließt das Bandteil 1 eine Durchtrittsöffnung 4 für das den Körperkanal umgebende Körpergewebe 2.

In der Hohlkammer 3 befindet sich ein Arbeitsfluid, insbesondere eine Flüssigkeit, z.B. Kochsalzlösung. Die Größe der Durchtrittsöffnung 4 hängt von der Menge des Arbeitsfluids in der Hohlkammer 3 ab. Durch Einbringen von Arbeitsfluid in die Hohlkammer 3 kann die Durchtrittsöffnung 4 verkleinert werden. Hierbei wird ein flexibler Innenabschnitt 1a des Bandteils 1, welcher zur Längsmittelachse 5 der Durchtrittsöffnung 4 benachbart ist, in Richtung zur Längsmittelachse 5 verschoben, wie dies bekannt ist. Durch Abführen von Arbeitsfluid aus der Hohlkammer 3 kann die Durchtrittsöffnung 4 wieder vergrößert werden.

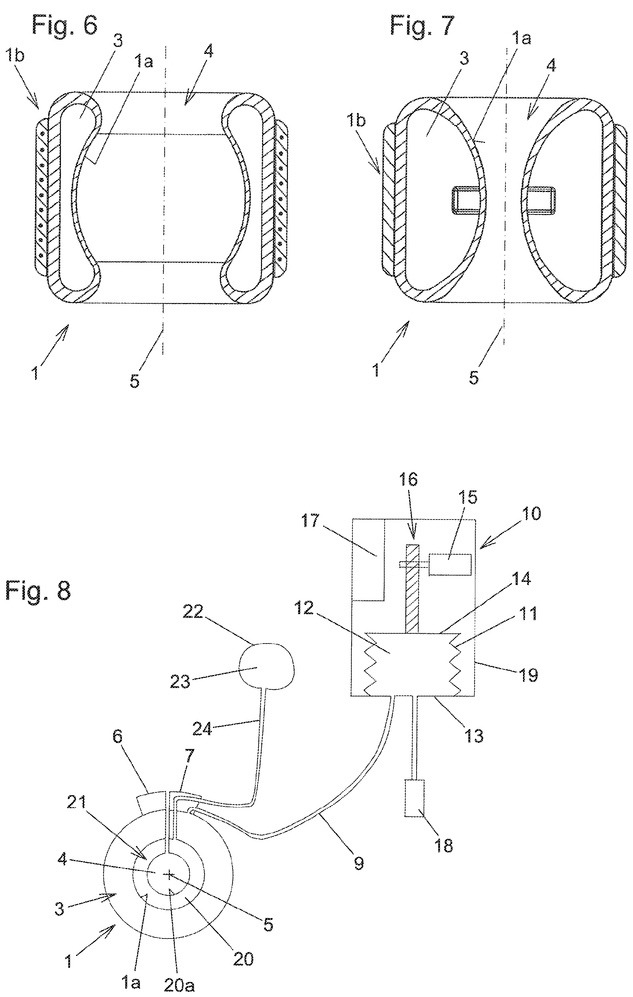

Fig. 6 zeigt den Zustand, in welchem die Durchtrittsöffnung 4 am größten ist (wobei der Druck des Arbeitsfluids in der Hohlkammer 3 dem Umgebungsdruck entspricht). Fig. 7 zeigt einen mit dem Arbeitsfluid befüllten Zustand, insbesondere dem maximal mit Arbeitsfluid befülltem Zustand (wobei der Druck des Arbeitsfluids in der Hohlkammer 3 über dem Umgebungsdruck liegt). In Fig. 7 sind Faltenbildungen, wie sie sich insbesondere ergeben würden, wenn das Bandteil nicht um den Harnleiter gelegt ist, nicht dargestellt. Ein von der Längsmittelachse 5 abgelegener Rückenabschnitt 1b des Bandteils 1 kann gegenüber dem Innenabschnitt 1a steif ausgebildet sein, insbesondere mittels einer Verstärkungslage, wodurch eine Verformung des Rückenabschnitts 1b zumindest weitgehend vermieden werden kann.

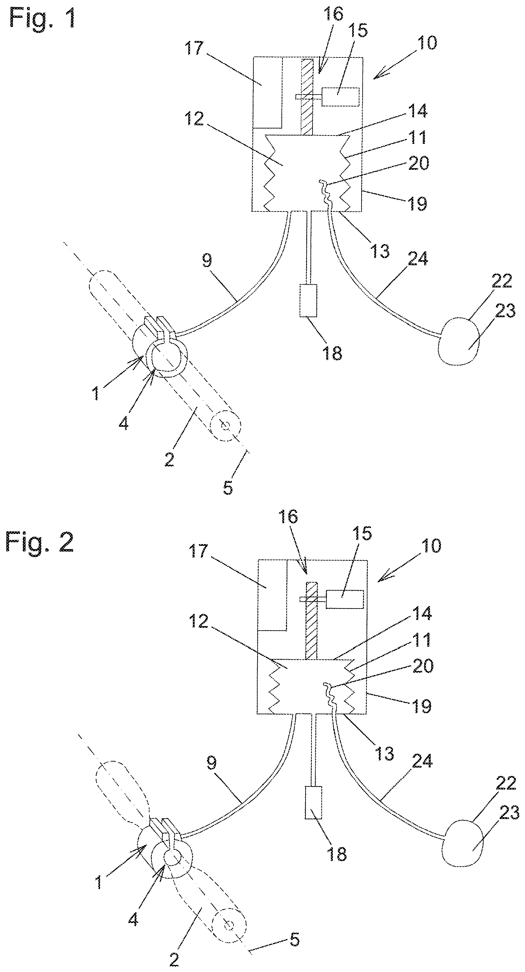

Im geschlossenen Zustand des um den Körperkanal gelegten Bandteils 1 kann dieses also einen Freigabezustand, in welchem der Körperkanal geöffnet ist (vgl. Fig. 1), und einen Absperrzustand, in welchem der Körperkanal geschlossen ist (vgl. Fig. 2), einnehmen. Im Freigabezustand kann der Druck des Arbeitsfluids in der Hohlkammer 3 beispielsweise dem Atmosphärendruck entsprechen. Im Absperrzustand wird die Hohlkammer 3 mit einer solchen Menge von Arbeitsfluid befüllt, dass der Körperkanal geschlossen ist.

Unterschiedliche Modifikationen der Ausbildung des Bandteils sind denkbar und möglich, so wäre es beispielsweise möglich, spezielle am Bandteil 1 angebrachte Verschlussteile überhaupt wegzulassen und die beiden Enden des Bandteils miteinander zu vernähen.

Das Bandteil 1 kann in bekannter Weise aus Silikon bestehen. Auch andere körperverträgliche Materialien sind grundsätzlich einsetzbar.

Im Ausführungsbeispiel ist an einem der Verschlussteile ein Anschlussstutzen 8 angeformt, dessen Hohlraum über einen durch das Verschlussteil laufenden Kanal mit der Hohlkammer 3 in Verbindung steht. Ein solcher Anschlussstutzen könnte auch an einer anderen Stelle des Bandteils vorgesehen sein. Eine als Schlauch ausgebildete Arbeitsfluidleitung 9 ist am Anschlussstutzen 8 angeschlossen.

Das Bandteil 1 steht über den Kanal der Arbeitsfluidleitung 9 mit einem vom Bandteil 1 örtlich getrennten Pumpteil 11 einer Pumpeinheit 10 in Verbindung, vgl. Fig. 1 bis 3. Mittels der Pumpeinheit 10 kann die Menge von Arbeitsfluid in der Hohlkammer 3 des Bandteils 1 verändert werden.

Ein Aufnahmeraum zur Aufnahme des Arbeitsfluids (= Arbeitsfluid-Aufnahmeraum) der medizinischen Einrichtung umfasst den gesamten zusammenhängenden Innenhohlraum der Einrichtung, in welchem sich im Betrieb Arbeitsfluid befindet. Die Hohlkammer 3 des Bandteils 1, und der durch das Verschlussteil verlaufende Kanal des Verschlussteils des Bandteils 1, sowie der Kanal der Arbeitsfluidleitung bilden somit jeweils einen Teil des Aufnahmeraums für das Arbeitsfluid. Das Pumpteil 11 der Pumpeinheit 10 besitzt im Ausführungsbeispiel einen mit Arbeitsfluid befüllten Innenraum 12, der ebenfalls einen Teil des Aufnahmeraums der Einrichtung bildet.

Zum Befüllen des Aufnahmeraums der medizinischen Einrichtung mit Arbeitsfluid ist in herkömmlicher Weise ein Port 18 vorhanden. Dieser kann beispielsweise über einen Schlauch an das Pumpteil 11 angeschlossen sein.

In den Ausführungsbeispielen wird das Pumpteil 11 von einem Balg gebildet, der von einem Bodenteil 13 und einem Deckelteil, welches ein Stellelement 14 darstellt, verschlossen ist. Ein elektrischer Antrieb 15 wirkt über ein Getriebe 16, beispielsweise einen Gewindetrieb, auf das Stellelement 14 ein, um das Volumen des Innenraums 12 zu ändern. Das Getriebe 16 ist günstigerweise selbsthemmend ausgebildet, sodass eine einmal eingestellte Position des Stellelements 14 ohne Zufuhr von elektrischer Energie an den Antrieb 15 gehalten wird.

Das Pumpteil 11 bildet im Ausführungsbeispiel also gleichzeitig ein Reservoir für das Arbeitsfluid, mit welchem die Hohlkammer 3 des Bandteils 1 zum Schließen des Körperkanals befüllt wird. Das Pumpteil 11 könnte beispielsweise auch von einer Kolben-Zylinder-Einheit gebildet werden, wobei das Stellelement 14 vom Kolben dieser Kolben-Zylinder-Einheit gebildet würde.

Der elektrische Antrieb 15 wird von einer Steuerelektronik 17 der Pumpeinheit 10 angesteuert, welche auch eine nicht dargestellte Batterie zur Speisung des Antriebs 15 mit elektrischem Strom aufweist. Die Bedienung der Steuerelektronik 17 durch den Benutzer erfolgt über eine geeignete, nicht näher dargestellte Benutzerschnittstelle. Bei der Benutzerschnittstelle kann es sich um eine per Kabel oder über Funk mit der Steuerelektronik 17 verbundene Bedieneinheit mit entsprechenden Schaltern handeln.

Die Benutzerschnittstelle kann außerhalb des Körpers angeordnet sein. Eine Implantation der Benutzerschnittstelle ist denkbar und möglich. Eine separate Benutzerschnittstelle könnte grundsätzlich auch entfallen, wobei mindestens ein vom Benutzer betätigbares Bedienelement an der Pumpeinheit 10 anzuordnen wäre. Dieses müsste entsprechend von außerhalb des Körpers betätigbar sein.

Die Komponenten der Pumpeinheit 10 sind in einem Gehäuse 19 angeordnet. Das Gehäuse 19 besteht aus einem körperverträglichen Material oder ist von einem solchen umhüllt.

Die medizinische Einrichtung weist im Weiteren einen Hilfsfluidbehälter 22 mit einer im Betrieb der Einrichtung mit Hilfsfluid befüllten Speicherkammer 23 auf. Der Hilfsfluidbehälter 22 ist günstigerweise in den Körper implantiert. Der Hilfsfluidbehälter 22 weist eine die Speicherkammer 23 begrenzende flexible Wand auf. Diese kann zumindest im Wesentlichen undehnbar ausgebildet sein.

Als "im Wesentlichen undehnbar" wird die Materialeigenschaft der Wand des Hilfsfluidbehälters 22 bezeichnet, bei welcher das Volumen der Speicherkammer 23 ausgehend von einem maximal entfalteten Zustand bei einer Erhöhung des Drucks des Hilfsfluids im Hilfsfluidbehälter 22 um 0,1 bar um weniger als 10%, vorzugsweise um weniger als 5% zunimmt. Der Hilfsfluidbehälter 22 könnte eine Armierung aufweisen, welche günstigerweise einen Elastizitätsmodul von zumindest 1000 N/mm², vorzugsweise von zumindest 5000 N/mm², aufweist.

Die Wand des Hilfsfluidbehälters 22 ist im Ausführungsbeispiel auch biegeschlaff ausgebildet, d.h. die Wand kehrt nicht von selbst zu einer früheren Form zurück. Die Wand des Hilfsfluidbehälters 22 könnte hierbei z.B. von einer Kunststofffolie gebildet sein. Der Hilfsfluidbehälter 22 gemäß den gezeigten Ausführungsbeispielen ist somit günstigerweise zusammenfaltbar, wobei das Volumen der Speicherkammer 23 durch ein Zusammenfalten oder Auseinanderfalten des Hilfsfluidbehälters 22 in Abhängigkeit von einem Umgebungsdruck (= Körperinnendruck) veränderbar ist.

Günstigerweise entspricht der Druck des Hilfsfluids bei Betrieb der Einrichtung dem Umgebungsdruck (= Körperinnendruck).

Die Wand des Hilfsfluidbehälters 22 besteht günstigerweise aus einem körperverträglichen Material, z.B. Polyamid oder Silikon, oder ist von einem solchen umhüllt. Die gegebenenfalls vorhandene Armierung kann in dieses Material eingebettet sein.

Die Speicherkammer 23 des Hilfsfluidbehälter 22 ist mittels einer Hilfsfluidleitung 24 mit einer Expansionskammer 21 eines Expansionskörpers 20 fluidleitend verbunden. Die Speicherkammer 23, der innere Kanal der Hilfsfluidleitung 24 und die Expansionskammer 21 bilden jeweils einen Teil eines Hilfsfluid-Aufnahmeraums der medizinischen Einrichtung. Als Hilfsfluid-Aufnahmeraum wird der gesamte Innenhohlraum der Einrichtung bezeichnet, in welchem sich im Betrieb der Einrichtung Hilfsfluid befindet.

Das vom Arbeitsfluid getrennte Hilfsfluid könnte z.B. über einen nicht näher dargestellten Port in die Hilfsfluidleitung 24 oder direkt in die Speicherkammer 23 eingebracht werden. Beim Hilfsfluid handelt es sich günstigerweise um eine Flüssigkeit, z.B. Kochsalzlösung.

Der Expansionskörper 20 ist im in den Figuren 1 bis 7 dargestellten, nicht erfindungsgemäßen, Beispiel einer medizinischen Einrichtung im mit Arbeitsfluid gefüllten Aufnahmeraum der Einrichtung angeordnet, nämlich im Innenraum 12 des Pumpteils 11.

Das Volumen der Expansionskammer 21 ist durch Einbringen von Hilfsfluid in die Expansionskammer 21 durch Entfalten oder Dehnen des Expansionskörpers 20, vergrößerbar. Wenn die Vergrößerung rein durch Entfalten erfolgt, kann der Expansionskörper 20 aus einem undehnbaren Material bestehen, sodass das maximale Volumen der Expansionskammer 21 begrenzt ist. Somit ist eine Druckerhöhung des Arbeitsfluids (wie weiter unten erläutert) durch die Begrenzung des Volumens der Expansionskammer 21 begrenzt. Andererseits kann das Volumen der Expansionskammer 21 durch eine elastisch dehnbare Ausbildung des Expansionskörpers 20 vergrößerbar sein.

Der Expansionskörper 20 kann auch biegeschlaff ausgebildet sein, d.h. der Expansionskörper 20 ist dann formlabil, wie dies in den Fig. 1 und 2 angedeutet ist.

Zumindest in dem in Fig. 2 dargestellten Absperrzustand des Bandteils 1 steht das Hilfsfluid unter einem geringeren Druck als das Arbeitsfluid. Die Expansionskammer 21 des Expansionskörpers 20 ist vorzugsweise vollständig zusammengefaltet. Damit befindet sich zumindest im Wesentlichen kein Hilfsfluid in der Expansionskammer 21. In anderen Worten ist das Hilfsfluid somit aufgrund des auf den Expansionskörper 20 einwirkenden höheren Drucks des Arbeitsfluids aus der Expansionskammer 21 heraus in die Speicherkammer 23 des Hilfsfluidbehälters 22 verdrängt. Das Volumen der Expansionskammer 21 ist somit zumindest im Wesentlichen gleich Null. Dies könnte in einer modifizierten Ausführungsform grundsätzlich auch anders sein.

Auch in Fig. 1, die sich auf den Freigabezustand des Bandteils 1 bezieht, ist die Expansionskammer 21 vollständig zusammengefaltet dargestellt, also mit einem Volumen von im Wesentlichen gleich Null. Dies ist dann der Fall, wenn das Hilfsfluid unter einem geringeren Druck als das Arbeitsfluid steht und/oder wenn das Hilfsfluid bei Gleichheit des Drucks (des Hilfsfluids und des Arbeitsfluids) durch die Elastizität des Expansionskörpers 20 aus der Expansionskammer 21 heraus verdrängt wird. Auch ein gewisses Restvolumen der Expansionskammer 21 im Freigabezustand kann vorhanden sein.

Steigt der Druck im Körperinneren (= Körperinnendruck) an, z.B. aufgrund eines Hustenanfalls, so wirkt eine durch den Körperinnendruck hervorgerufene Kraft direkt auf den Hilfsfluidbehälter 22 ein. Beim Ausüben einer Kraft auf den Hilfsfluidbehälter 22 erhöht sich der Druck des Hilfsfluids entsprechend. Ist der Körperinnendruck, und damit der Druck des Hilfsfluids, größer als der Druck des Arbeitsfluids, so wird der Hilfsfluidbehälter 22 komprimiert und Hilfsfluid wird aus der Speicherkammer 23 in die Expansionskammer 21 verdrängt. Falls der Expansionskörper 20 der Expansion der Expansionskammer 21 eine elastische Rückstellkraft entgegensetzt, kann auch diese durch den Druck des Hilfsfluids überwunden werden (Der Expansionskörper 20 wird somit erst expandiert, wenn der Umgebungsdruck den Druck des Arbeitsfluids so weit übersteigt, dass auch die elastische Rückstellkraft durch den Druck des Hilfsfluids überwunden wird). Die resultierende Vergrößerung des Volumens der Expansionskammer 21 führt dabei zu einer Erhöhung des Drucks im Innenraum 12 des Pumpteils 11. Dabei wird Arbeitsfluid aus dem Innenraum 12 ausgestoßen und in die Hohlkammer 3 des Bandteils 1 eingebracht. Der Innenabschnitt 1a des Bandteils 1 wird in Richtung hin zur Längsmittelachse 5 verschoben und dabei eine Zusatzkraft auf das durch die Durchtrittsöffnung 4 geführte Körpergewebe 2 aufgebracht. Dieser Zustand des Bandteils 1 wird in dieser Schrift als Stresszustand des Bandteils 1 bezeichnet und ist in Fig. 3 dargestellt.

Verringert sich in der Folge der Körperinnendruck und damit der Druck des Hilfsfluids wieder, sodass der Druck des Hilfsfluids kleiner als der Druck des Arbeitsfluids ist (gegebenenfalls zuzüglich der elastischen Rückstellkraft des Expansionskörpers), so vergrößert sich das Volumen der Speicherkammer 23 des Hilfsfluidbehälters 22 unter Aufnahme von aus der Expansionskammer 21 des Expansionskörpers 20 abgeführtem Hilfsfluid. Nach dem Stressereignis befindet sich das Bandteil 1 somit wiederum im Absperrzustand, vgl. Fig. 2.

Wenn der Körperkanal ausgehend vom Absperrzustand des Bandteils 1, z.B. zum Ablassen von Urin, geöffnet werden soll, d.h. das Bandteil 1 durch Verlagern von Arbeitsfluid aus der Hohlkammer 3 in den Innenraum 12 des Pumpteils 11 den Freigabezustand (siehe Fig. 1) einnimmt und auch kein Stressereignis vorliegt, bleibt das Volumen der Expansionskammer 21 im Wesentlichen gleich Null oder relativ gering. Somit muss beim Verstellen des Bandteils 1 vom Absperrzustand in den Freigabezustand (und umgekehrt) kein oder nur wenig Hilfsfluid von der Expansionskammer 21 in die Speicherkammer 23 des Hilfsfluidbehälters 22 gefördert werden. Die Menge an zu verschiebendem Arbeitsfluid kann dadurch minimiert und der damit verbundene Energieaufwand zum Verschieben von Arbeitsfluid vermindert werden. Das Intervall zum Aufladen oder Austauschen der Batterie der Steuerelektronik 17 kann dadurch verlängert werden.

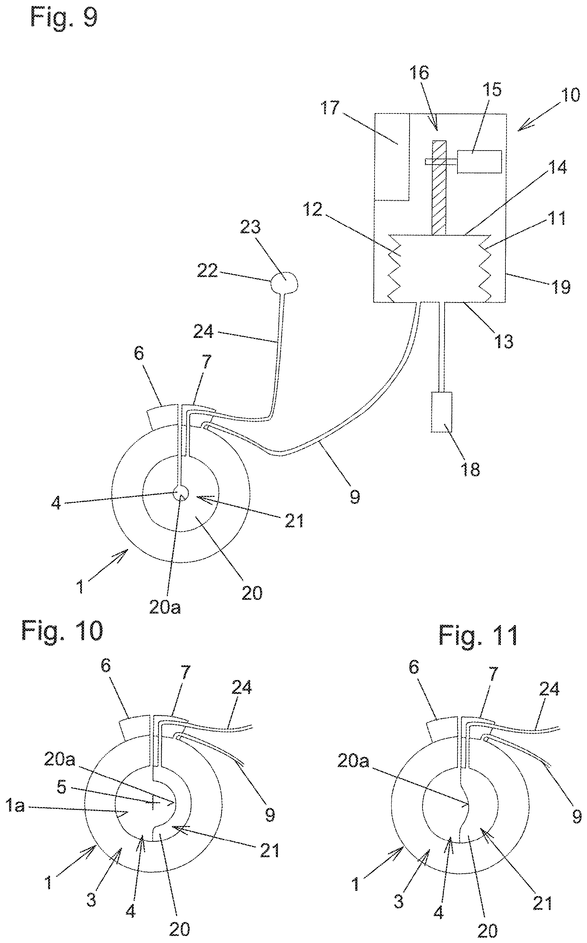

In den Fig. 8 und 9 ist ein Ausführungsbeispiel einer medizinischen Einrichtung gemäß der Erfindung dargestellt. Der Aufbau der Pumpeinheit 10 und des Hilfsfluidbehälters 22 entspricht jenem des zuvor erläuterten Beispiels einer medizinischen Einrichtung, sodass in den Erläuterungen zum erfindungsgemäßen Ausführungsbeispiel hauptsächlich auf die Unterschiede zum zuvor erläuterten Beispiel hingewiesen wird. Abgesehen von den im Folgenden angeführten Unterschieden gelten die Erläuterungen zum zuvor erläuterten Beispiel auch beim erfindungsgemäßen Ausführungsbeispiel. So wird auch hinsichtlich der Ausbildung der in Fig. 8 und 9 nur schematisch dargestellten Verschlussteile 6, 7 des Bandteils 1 auf die Erläuterungen zum zuvor erläuterten Beispiel verwiesen.

Bei der medizinischen Einrichtung gemäß dem erfindungsgemäßen Ausführungsbeispiel ist vorgesehen, dass der Expansionskörper 20 an einer der Durchtrittsöffnung 4 zugewandten Seite des Bandteils 1, also am Innenabschnitt 1a am Bandteil 1 angeordnet ist. Dies ist in den Fig. 8 und 9 schematisch dargestellt, wobei sich die Expansionskammer 21 in Richtung der Längserstreckung des Bandteils 1 erstreckt, im Wesentlichen über die gesamte Länge des Bandteils 1. Der Expansionskörper 20 ist also im erfindungsgemäßen Ausführungsbeispiel schlauchartig ausgebildet, mit beidseitig verschlossenen Enden.

Der am Bandteil 1 angeordnete Expansionskörper 20 weist im geschlossenen Zustand des Bandteils 1 bezogen auf die Umfangsrichtung der Längsmittelachse 5 eine im Wesentlichen umlaufende Anlagefläche 20a zur Anlage am Körpergewebe 2 auf.

Im Absperrzustand des geschlossenen Bandteils 1 wird die Hohlkammer 3 mit einer solchen Menge von Arbeitsfluid befüllt, dass der Körperkanal geschlossen ist, vgl. Fig. 8.

Wenn Arbeitsfluid abgelassen wird, nimmt das Bandteil 1 den nicht gesondert dargestellten Freigabezustand ein, in welchem der Körperkanal geöffnet ist.

Steigt der Druck im Körperinneren (= Körperinnendruck) ausgehend vom in Fig. 8 dargestellten Absperrzustand des Bandteils 1 bei Vorliegen eines Stressereignisses an, so wirkt dieser Körperinnendruck analog zum zuvor erläuterten Beispiel auf den Hilfsfluidbehälter 22 ein. Ist der Körperinnendruck, und damit der Druck des Hilfsfluids, größer als der vom Körpergewebe 2 ausgeübte Gegendruck auf den Expansionskörper 20, so wird der Hilfsfluidbehälter 22 komprimiert und Hilfsfluid aus der Speicherkammer 23 in die Expansionskammer 21 des Expansionskörpers 20 geleitet. Falls der Expansionskörper 20 der Expansion der Expansionskammer 21 eine elastische Rückstellkraft entgegensetzt, kann auch diese durch den Druck des Hilfsfluids überwunden werden. Durch die Vergrößerung des Volumens der Expansionskammer 21 wirkt der Expansionskörper 20 direkt komprimierend auf das den Körperkanal umgebende Körpergewebe 2 ein, um ein zuverlässiges Absperren des Körperkanals während eines Stressereignisses zu gewährleisten, vgl. den in Fig. 9 dargestellten Stresszustand des Bandteils 1, bei welchem eine Zusatzkraft auf das durch die Durchtrittsöffnung 4 geführte Körpergewebe 2 aufgebracht ist. Das Körpergewebe 2 selbst ist in den Fig. 8 und 9 nicht eingezeichnet.

Erreicht der Körperinnendruck wieder einen Grundzustand, d.h. ohne dass ein Stressereignis vorliegt, strömt das Hilfsfluid wiederum aus der Expansionskammer 21 in den Hilfsfluidbehälter 22 zurück, vgl. den in Fig. 8 dargestellten Absperrzustand des Bandteils 1.

Beim erfindungsgemäßen Ausführungsbeispiel erfolgt das Zurückströmen des Hilfsfluids in den Hilfsfluidbehälter 22 somit durch Einwirken des vom Körpergewebe 2 ausgeübten Gegendruckes zusammen mit dem von der mit Arbeitsfluid gefüllten Hohlkammer 3 auf den Expansionskörper 20 ausgeübten Druck. Falls der Expansionskörper 20 der Expansion der Expansionskammer 21 eine elastische Rückstellkraft entgegensetzt, kann diese beim Zurückströmen des Hilfsfluids unterstützend wirken.

Beim erfindungsgemäßen Ausführungsbeispiel kann vorgesehen sein, dass der Expansionskörper 20 und das Bandteil 1 materialeinstückig ausgebildet sind. Günstigerweise ist vorgesehen, dass der Expansionskörper 20 im Ausführungsbeispiel elastisch dehnbar ausgebildet ist. In einer anderen Ausführungsform könnte aber auch vorgesehen sein, dass der Expansionskörper 20 im Wesentlichen undehnbar und/oder biegeschlaff ausgebildet ist.

Das Bandteil 1 und/oder der Expansionskörper 20 könnte(n) im Ausführungsbeispiel aus einem körperverträglichen Material, z.B. aus Silikon, bestehen.

Im Ausführungsbeispiel ist am Verschlussteil 7 ein nicht näher dargestellter Anschlussstutzen zur Verbindung des Bandteils 1 mit der Hilfsfluidleitung 24 angeformt. Der Innenraum des Anschlussstutzens steht über einen durch das Verschlussteil 7 laufenden Kanal mit der Expansionskammer 21 in Verbindung. Ein solcher Anschlussstutzen könnte auch an einer anderen Stelle des Expansionskörpers 20 oder des Bandteils 1 vorgesehen sein.

In den Fig. 10 und 11 ist eine Variante des Bandteils 1 gemäß des zweiten Ausführungsbeispiels dargestellt, wobei im Folgenden insbesondere auf die Unterschiede zum in Fig. 8 und 9 dargestellten Bandteil 1 eingegangen wird.

In der in Fig. 10 und 11 gezeigten Abwandlungsform des Bandteils 1 erstreckt sich der Expansionskörper 20 in Richtung der Längserstreckung des Bandteils 1 über ca. die Hälfte der Länge des Bandteils 1. Die Durchtrittsöffnung 4 ist im in Fig. 10 dargestellten Absperrzustand des Bandteils 1 im Unterschied zum zweiten Ausführungsbeispiel nicht kreisrund. Die Anlagefläche 20a des Expansionskörpers 20 erstreckt sich im geschlossenen Zustand des Bandteils 1 bezogen auf eine Umfangsrichtung ausgehend von der Längsmittelachse 5 über 180° des Umfangs der Durchtrittsöffnung 4.

Ansonsten verhält sich das Bandteil 1 gemäß der in Fig. 10 und 11 gezeigten Abwandlungsform beim Auftreten eines Stressereignisses analog zum Bandteil 1 des zweiten Ausführungsbeispiels der medizinischen Einrichtung, weshalb auf die entsprechenden Erläuterungen zum zweiten Ausführungsbeispiel verwiesen wird.

Abgesehen von der in den Ausführungsbeispielen gezeigten Pumpeinheit könnte die medizinische Einrichtung grundsätzlich auch in Kombination mit einer im Stand der Technik hinlänglich bekannten, beispielsweise manuell betätigbaren, Pumpeinheit verwendet werden.

### Legende

### zu den Hinweisziffern:

- 1: Bandteil
- 1a: Innenabschnitt
- 1b: Rückenabschnitt
- 2: Körpergewebe
- 3: Hohlkammer
- 4: Durchtrittsöffnung
- 5: Längsmittelachse
- 6: erstes Verschlussteil
- 6a: Einstecköffnung
- 7: zweites Verschlussteil
- 7a: Zunge
- 8: Anschlussstutzen
- 9: Arbeitsfluidleitung
- 10: Pumpeinheit
- 11: Pumpteil
- 12: Innenraum
- 13: Bodenteil
- 14: Stellelement
- 15: Antrieb
- 16: Getriebe
- 17: Steuerelektronik
- 18: Port
- 19: Gehäuse
- 20: Expansionskörper
- 20a: Anlagefläche
- 21: Expansionskammer
- 22: Hilfsfluidbehälter
- 23: Speicherkammer
- 24: Hilfsfluidleitung

## Patentansprüche

1. Medizinische Einrichtung zum Absperren eines Körperkanals, umfassend
- ein Bandteil (1), das um das den Körperkanal umgebende Körpergewebe (2) legbar ist und zu einem eine Durchtrittsöffnung (4) für das Körpergewebe (2) umschließenden Ring schließbar ist und das eine Hohlkammer (3) aufweist, die einen Teil eines Aufnahmeraums der Einrichtung zur Aufnahme von Arbeitsfluid darstellt, und
- eine Pumpeinheit (10) zum Fördern des Arbeitsfluids, wobei durch Einbringen des Arbeitsfluids in die Hohlkammer (3) die Durchtrittsöffnung (4) verkleinerbar ist, wobei die Einrichtung im Weiteren einen Expansionskörper (20) mit einer Expansionskammer (21) aufweist, und durch Einbringen eines vom Arbeitsfluid getrennten Hilfsfluid in die Expansionskammer (21) die Expansionskammer (21) vergrößerbar ist, **dadurch gekennzeichnet, dass** der Expansionskörper (20) an einer der Durchtrittsöffnung (4) zugewandten Seite des Bandteils (1) am Bandteil (1) angeordnet ist und dass die medizinische Einrichtung dazu ausgebildet ist, die Expansionskammer mit Hilfsfluid zu befüllen und so durch die dadurch bewirkte Vergrößerung des Volumens der Expansionskammer direkt komprimierend auf das den Körperkanal umgebende Körpergewebe einwirkt, um ein zuverlässiges Absperren des Körperkanals während einer Erhöhung des Körperinnendrucks während eines Stressereignisses zu gewährleisten.

2. Medizinische Einrichtung nach Anspruch 1, **dadurch gekennzeichnet, dass** die Einrichtung einen Hilfsfluidbehälter (22) mit einer Speicherkammer (23) für das Hilfsfluid aufweist, deren Volumen in Abhängigkeit von einem Umgebungsdruck veränderbar ist, und der Hilfsfluidbehälter (22) über eine Hilfsfluidleitung (24) mit der Expansionskammer (21) verbunden ist.

3. Medizinische Einrichtung nach Anspruch 2, **dadurch gekennzeichnet, dass** der Hilfsfluidbehälter (22) eine die Speicherkammer (23) begrenzende flexible Wand aufweist.

4. Medizinische Einrichtung nach Anspruch 3, **dadurch gekennzeichnet, dass** die Wand im Wesentlichen undehnbar ist.

5. Medizinische Einrichtung nach Anspruch 3 oder 4, **dadurch gekennzeichnet, dass** die Wand biegeschlaff ist.

6. Medizinische Einrichtung nach einem der Ansprüche 1 bis 5, **dadurch gekennzeichnet, dass** die Pumpeinheit (10) ein Pumpteil (11) aufweist, das einen Innenraum (12) zur Aufnahme von Arbeitsfluid aufweist, wobei das Volumen des Innenraums (12) mittels eines Antriebs (15) veränderbar ist.

7. Medizinische Einrichtung nach einem der Ansprüche 1 bis 6, **dadurch gekennzeichnet, dass** der am Bandteil (1) angeordnete Expansionskörper (20) eine Anlagefläche (20a) zur Anlage am Körpergewebe (2) aufweist.

8. Medizinische Einrichtung nach einem der Ansprüche 1 bis 7, **dadurch gekennzeichnet, dass** das Volumen der Expansionskammer (21) bei einem im Vergleich zum Druck des Hilfsfluids unter einem höheren Druck stehenden Arbeitsfluid zumindest im Wesentlichen gleich Null ist.

9. Medizinische Einrichtung nach einem der Ansprüche 2 bis 8, **dadurch gekennzeichnet, dass** sich der Druck des Hilfsfluids beim Ausüben einer Kraft auf den Hilfsfluidbehälter (22) erhöht.

## Claims

1. Medical device for shutting off an anatomical channel, comprising
- a band part (1) which can be placed around the body tissue (2) surrounding the anatomical channel and which can be closed to form a ring that encloses a through-opening (4) for the body tissue (2), and which has a hollow chamber (3) constituting a part of a receiving space of the device for receiving working fluid , and
- a pump unit (10) which serves to convey the working fluid, wherein the through-opening (4) can be made smaller by introducing the working fluid into the hollow chamber (3),
wherein the device moreover has an expansion body (20) with an expansion chamber (21), and the expansion chamber (21) can be made larger by introducing an auxiliary fluid, separate from the working fluid, into the expansion chamber (21), **characterised in that** the expansion body (20) is arranged at the band part(1), on a side of the band part (1) directed toward the through-opening (4), and **in that** the medical device is embodied to fill the auxiliary fluid into the expansion chamber and the resulting enlargement of the volume of the expansion chamber thereby acting directly compressing onto the body tissue surrounding the anatomical channel, in order to ensure a reliable shutting-off of the body channel during an increase in internal body pressure occurring in a stress event.

2. Medical device according to claim 1, **characterized in that** the device has an auxiliary fluid container (22) with a storage chamber (23) for the auxiliary fluid, the volume of which storage chamber (23) is variable depending on an ambient pressure, and the auxiliary fluid container (22) is connected to the expansion chamber (21) via an auxiliary fluid line (24).

3. Medical device according to claim 2, **characterized in that** the auxiliary fluid container (22) has a flexible wall delimiting the storage chamber (23).

4. Medical device according to claim 3, characterized m that the wall is substantially non-extensible.

5. Medical device according to claim 3 or 4, **characterized in that** the wall is pliable.

6. Medical device according to one of claims 1 through 5, **characterized in that** the pump unit (10) has a pump part (11) which has an interior (12) for receiving working fluid, wherein the volume of the interior (12) is variable by a drive (15).

7. Medical device according to one of claims 1 through 6, **characterized in that** the expansion body (20) arranged at the band part (I) has a bearing surface (20a) for bearing on the body tissue (2).

8. Medical device according to one of claims 1 through 7, **characterized in that** the volume of the expansion chamber (21) is at least substantially equal to zero when a working fluid is at a higher pressure compared to the pressure of the auxiliary fluid .

9. Medical device according to one of claims 2 through 8, **characterized in that** the pressure of the auxiliary fluid rises when a force is exerted on the auxiliary fluid container (22).

## Revendications

1. Dispositif médical destiné à obturer un canal corporel, comprenant
- un élément en forme de bande (1) qui peut être disposé autour du tissu corporel (2) entourant le canal corporel, et qui peut être fermé pour former un anneau entourant une ouverture de passage (4) pour le tissu corporel (2), et qui présente une chambre creuse (3) constituant une partie d'un espace de réception du dispositif destiné à recevoir un fluide de travail, et
- une unité de pompe (10) destinée à déplacer le fluide de travail, l'introduction du fluide de travail dans la chambre creuse (3) permettant de réduire l'ouverture de passage (4), le dispositif présentant en outre un corps d'expansion (20) comportant une chambre d'expansion (21), et l'introduction d'un fluide auxiliaire séparé du fluide de travail dans la chambre d'expansion (21) permettant d'agrandir la chambre d'expansion (21), **caractérisé en ce que** le corps d'expansion (20) est disposé sur l'élément en forme de bande (1) du côté de celui-ci tourné vers l'ouverture de passage (4), et **en ce que** le dispositif médical est conçu pour remplir la chambre d'expansion avec le fluide auxiliaire et ainsi, par l'augmentation du volume de la chambre d'expansion qui en résulte, agir directement de manière compressive sur le tissu corporel entourant le canal corporel, afin d'assurer une obturation fiable du canal corporel lors d'une augmentation de la pression interne du corps au cours d'un événement de stress.

2. Dispositif médical selon la revendication 1, **caractérisé en ce que** le dispositif comporte un réservoir de fluide auxiliaire (22) présentant une chambre de stockage (23) pour le fluide auxiliaire et dont le volume peut être modifié en fonction d'une pression ambiante, et le réservoir de fluide auxiliaire (22) est relié à la chambre d'expansion (21) par une conduite de fluide auxiliaire (24).

3. Dispositif médical selon la revendication 2, **caractérisé en ce que** le réservoir de fluide auxiliaire (22) présente une paroi flexible délimitant la chambre de stockage (23).

4. Dispositif médical selon la revendication 3, **caractérisé en ce que** la paroi est sensiblement inextensible.

5. Dispositif médical selon la revendication 3 ou 4, **caractérisé en ce que** la paroi est souple en flexion.

6. Dispositif médical selon l'une des revendications 1 à 5, **caractérisé en ce que** l'unité de pompe (10) comporte un élément de pompe (11) présentant un espace intérieur (12) destiné à recevoir le fluide de travail, le volume de l'espace intérieur (12) pouvant être modifié au moyen d'un entraînement (15).

7. Dispositif médical selon l'une des revendications 1 à 6, **caractérisé en ce que** le corps d'expansion (20) disposé sur l'élément en forme de bande (1) présente une surface d'appui (20a) destinée à venir en appui contre le tissu corporel (2).

8. Dispositif médical selon l'une des revendications 1 à 7, **caractérisé en ce que** le volume de la chambre d'expansion (21) est au moins sensiblement égal à zéro lorsque le fluide de travail est soumis à une pression supérieure à la pression du fluide auxiliaire.

9. Dispositif médical selon l'une des revendications 2 à 8, **caractérisé en ce que** la pression du fluide auxiliaire augmente lorsqu'une force est exercée sur le réservoir de fluide auxiliaire (22).
